# EUROPÄISCHE PATENTSCHRIFT

(11) **EP 1 179 334 B1**
(45) Veröffentlichungstag und Bekanntmachung des Hinweises auf die Patenterteilung: **24.08.2005**
(21) Anmeldenummer: 01114538.0
(22) Anmeldetag: 16.06.2001
(51) Int. Cl.: A61K 7/06

(54) **Haartonikum zur Vorbeugung oder Behandlung von Haarausfall**
Hair tonic for preventing or treating hair loss
Composition tonifiante pour la prévention et le traitement de la perte de cheveux

(30) Priorität: 22.07.2000 DE 10035735
(43) Veröffentlichungstag der Anmeldung: 13.02.2002
(73) Patentinhaber: Wella Aktiengesellschaft, 64274 Darmstadt (DE)
(72) Erfinder: Noser, Friedrich, 1729 Bonnefontaine (CH); Hehner, Ursula, 64395 Brensbach (DE); Wagenknecht, Bernd, 55130 Mainz (DE)

(56) Entgegenhaltungen:
- EP-A- 0 116 439
- EP-A- 0 693 278
- DE-A- 3 143 626
- DE-A- 4 134 137
- GB-A- 2 163 460
- US-A- 5 116 607
- US-A- 5 215 759
- US-A- 5 523 078
- PATENT ABSTRACTS OF JAPAN vol. 014, no. 294 (C-0732), 26. Juni 1990 (1990-06-26) & JP 02 096581 A (SHISEIDO CO LTD), 9. April 1990 (1990-04-09)

## Beschreibung

Gegenstand der Erfindung ist ein kosmetisches Mittel, insbesondere ein Haartonikum zur Vorbeugung oder Behandlung von Haarausfall mit Gehalt an einer Wirkstoffkombination von Fettsäuren, Biotin und Coffein.

Das Wachstum von menschlichen Haaren unterliegt bekanntermaßen einem natürlichen Wachstumsrhythmus, bestehend aus einer Wachstumsphase (Anagen), einer Übergangsphase (Katagen) und einer Ruhephase (Telogen). Hierbei kommt es zu einem normalen, natürlichen Haarwechsel, da zu Beginn jedes Anagens das Kolbenhaar abgeworfen wird. Demzufolge fallen auf der behaarten Kopfhaut täglich 80 bis 100 Kolbenhaare aus. Diese Zahl ist ein Richtwert, welcher über kürzere Zeit auch nach oben und nach unten abweichen kann, ohne dass irgendwelche Befürchtungen für das natürliche Haarwachstum zu hegen sind. Übersteigen die täglich ausfallenden Mengen von Haar über längere Zeit deutlich die Norm, so liegt ein reversibler oder ein irreversibler Haarausfall vor. Der Anagenfollikel ist von Natur aus als hochaktives Organ sehr empfindlich gegen Störeinflüsse, wohingegen der Telogenfollikel kaum darauf reagiert. Die natürliche erste Reaktion des Haarfollikels auf deutliche Störeinflüsse ist deshalb die Flucht ins Telogen und ein erneutes Anagen, sobald die Störeinflüsse wegfallen. Massive Störeinflüsse führen von der Abnahme der Haarstärke bis zum Anagenhaarausfall, der auftritt, wenn dem Haarfollikel keine Zeit mehr für die Flucht ins Telogen bleibt. Es gibt keine strenge Beziehung zwischen dem Typ des Störeinflusses und der Reaktionsweise des Haarorgans.

Reversibler Haarausfall ist insgesamt recht verbreitet. Beispiele hierfür sind Haarausfall nach fiebrigen und zehrenden Krankheiten, Haarausfall infolge sehr einseitiger Ernährung, Haarausfall bei Eisenmangel, Haarausfall unter eiweissfreier Ernährung, Haarausfall nach Fastenkuren, Haarausfall nach Einnahme von Medikamenten gegen Krebs. Eine natürliche Form des Haarausfalles ist der Haarausfall bei Frauen nach der Geburt. Die Gestagene, Hormone, welche während der Schwangerschaft in grossen Mengen gebildet werden, synchronisieren die Wachstumszyklen der Kopfhaare. Resultat ist ein sehr hohes Verhältnis von Anagenhaaren zu Telogenhaaren. Nach der Geburt schlägt dieses Verhältnis ins Gegenteil um, das heisst, viele Anagenhaare wechseln ins Telogen hinüber. Nach einigen Monaten folgt ein Anagenschub, im Verlaufe dessen Kolbenhaare in Massen gleichzeitig bis zur auffälligen Lichtung des Haarbestandes ausfallen können. Haben die neuen Anagenhaare die angestammte Länge wieder erreicht, ist der ursprüngliche Zustand wieder erreicht. Haarausfall nach massivem psychischem Stress ist eine weitere Form reversiblen Haarausfalls. Wie dieser Haarausfall zustande kommt, ist unbekannt. Es scheint, dass Stress, der mannigfaltige Funktionen unseres Stoffwechsels nachhaltig beeinträchtigen kann, auch die Tätigkeit des ständig auf Maximalleistung laufenden Haarorgans in Mitleidenschaft zieht.

Die klassische Form des bisher noch weitgehend als irreversibel einzustufenden Haarausfalls ist der androgenetische Haarausfall, bzw. die Umwandlung von Terminalhaar in Vellushaar. Er setzt im frühen Mannesalter (ab rund 17 Jahren) ein und dürfte im Alter von rund 50 Jahren abgeschlossen sein. Er wird durch die Androgene ausgelöst. Die zwei wichtigsten biologisch aktiven Formen der Androgene sind das Testosteron und das von ihm abgeleitete 5-alpha-Dihydrotestosteron. Ein Enzym, die 5-alpha-Reduktase, führt das Testosteron in das Dihydrotestosteron über. Befunde weisen darauf hin, dass das Dihydrotestosteron die ausschlaggebende Hormonform für die Beeinflussung des Wachstums des Haares ist. Androgene sind, was die Wirkung auf Kopf- und Körperhaar anbelangt, Solisten im Hormonorchester. Merkwürdigerweise bewirken sie am Körperhaar genau das Gegenteil wie am Kopfhaar. Verwandeln sie auf der behaarten Kopfhaut Terminalhaare in kaum noch sichtbare Vellushaare, so veranlassen sie in der Bartregion die Vellushaare des Pubertierenden zur Produktion des Wahrzeichens der Männlichkeit. Im hohen Alter führt der Altershaarausfall, der vorwiegend die Follikeltransformation des Terminalfollikels in Richtung Intermediär- und Vellusfollikel beinhaltet, bei beiden Geschlechtern zu einer weiteren, indessen diffusen Lichtung des noch existierenden Haarbestandes. In dieser Altersstufe bedarf das Haar einer besonders angepassten und wirkungsvollen Pflege. Störungen, welche zur Zerstörung des Haarorgans führen, verursachen eine bleibende Kahlheit.

Die Aufgabe der vorliegenden Erfindung bestand darin, ein gesundes Haarwachstum und die Bildung von Haaren von natürlicher Schönheit zu fördern oder sicherzustellen, Haarveränderungen und Haarausfall beliebiger Ursache entgegenzuwirken, d.h. zu verhindern oder zumindest signifikant zu reduzieren, die Erholung bei reversiblen Haarausfällen zu fördern, und bei irreversiblem, insbesondere androgenetischem Haarausfall die Transformation so weit wie möglich zu bremsen, sie aufzuhalten oder im Idealfall auch umzukehren.

Die EP 0 116 439 beschreibt Haartonika mit einem Gehalt an bestimmten Fettsäuren als Wirkstoffen. Als Anwendungsgebiete werden die Verhinderung von Schuppen, die Verhinderung von Kopfhautjucken und eine Beschleunigung des Haarwachstums angegeben, was bei geschorenen Kaninchen untersucht wurde. Eine Wirkung hinsichtlich Verhinderung oder Reduzierung von Haarausfall beim Menschen wird nicht beschrieben.

DE-A1- 4 134 137 offenbart eine Haarboden-Bioaktivlotion, welche eine Verhinderung des Haarausfalls bewirkt.

Die Lotion besteht aus einem wäßrig-alkoholischen Komplex A und aus einem Ölkomplex B.

Die Bioaktivlotion enthält zusätzlich eine Kombination der Vitamine A,E,F, und H.

WO-A-9 831 326 beschreibt wäßrige Biotin-und Coffein enthaltende Liposome zusammensetzungen als Haarbehandlungs mittel.

EP-A-693 278 offenbart kosmetische Zusammensetzungen zur Verhinderung des Haarausfalls. Als Wirkstoffen werden C₁₂-C₁₄ Fettsäuren in Kombination mit Cumarin und Alvaloid verwendet.

Es wurde nun gefunden, dass eine gesteigerte Wirkung hinsichtlich gesundem Haarwachstum, Vorbeugung, Verhinderung oder Verminderung von Haarausfall unter Potenzierung der Effekte der Einzelsubstanzen, welche an verschiedenen, für das Haarwachstum essentiellen Stellen eingreifen, erreicht wird durch eine Kombination von bestimmten Fettsäuren, Biotin und Coffein.

Gegenstand der Erfindung ist daher ein kosmetisches Mittel, insbesondere ein Haartonikum mit einem Gehalt an
(A) mindestens einer Fettsäure oder deren Salzen, ausgewählt aus laurinsäure, Myristinsäure und Isomyristinsäure und
(B) Biotin und
(C) Coffein.

Ein weiterer Gegenstand der Erfindung ist die Verwendung einer Kombination der vorgenannten Komponenten (A), (B) und (C) zur Vorbeugung oder Behandlung von Haarausfall.

### Fettsaüre (A)

Die Fettsäure liegt vorzugsweise in einer Menge von 0,01 bis 10 Gew.%, besonders bevorzugt von 0,1 bis 2 Gew.% vor. Geeignet sind, Laurinsäure, Myristinsäure, Isomyristinsäure, oder deren kosmetisch akzeptable Salze. Besonders bevorzugt sind Laurinsäure und Myristinsäure oder eine Kombination dieser beiden Säuren. Kosmetisch akzeptable Salze sind beispielsweise Alkali-, Erdalkali oder Ammoniumsalze. Vorzugsweise liegt die Fettsäure aber nicht als Salz sondern in Form der freien Säure vor, welche eine höhere Wirksamkeit hat.

### Biotin

Biotin liegt erfindungsgemäß vorzugsweise in einer Menge von 0,001 bis 1 Gew.%, besonders bevorzugt von 0,005 bis 0,1 Gew.% vor. Biotin gehört zum Vitamin-B-Komplex (als B₇) und wird auch Vitamin H genannt. Die chemische Bezeichnung ist D-cis-Hexahydro-2-oxothieno-[3,4-d]-imidazol-4-valeriansäure. Die Verwendung von Biotin beeinflußt die Qualität der Haare positiv. Biotin ist das Vitamin der Keratinisierung, d.h. eine verbesserte Versorgung der Haarorgane mit Biotin verbessert die Keratinisierung der Haare. Biotin erhöht bei feinem Haar hoch signifikant und bei normalem Haar signifikant den Reisswiderstand, bzw. die Festigkeit und damit die Resistenz gegen Umwelteinflüsse und belastende Behandlungen. Biotin festigt die Verankerung der Kolbenhaare in der Kopfhaut. Folge ist eine Abnahme der Zahl ausfallender Haare bis zur Stabilisierung auf einem Minimum.

### Coffein

Coffein liegt erfindungsgemäß vorzugsweise in einer Menge von 0,01 bis 10 Gew.%, besonders bevorzugt von 0,1 bis 2 Gew.% vor. Coffein ist ein zu den Purinen zählendes Pflanzenalkaloid und wird auch als Thein, Methyltheobromin oder Guaranin bezeichnet. Die chemische Bezeichnung ist 1,3,7-Trimethylxanthin. Es handelt sich um einen Naturstoff, der sich in einer Reihe von Pflanzen findet, beispielsweise in Kaffeebohnen, schwarzem Tee, Mate-Tee, Kakaokernen und Kolanüssen. Coffein erweitert die Blutgefässe, verstärkt den Anstrom von Blut an das Haarorgan, erhöht damit dessen Nährstoffangebot und verlängert den Haarwachstumszyklus.

### Tonikum

Die Basis für das erfindungsgemäße Haartonikum kann eine üblicherweise für kosmetische Zusammensetzungen verwendete Basis sein, beispielsweise ein wässriges, alkoholisches oder ein wässrig-alkoholisches Medium mit vorzugsweise mindestens 10 Gew.% Wasser und mindestens 30 Gew.% Alkohol. Das Lösungsmittelsystem ist vorzugsweise in einer Menge von 70 bis 99,5, besonders bevorzugt von 90 bis 99 Gew.% enthalten. Als Alkohole können insbesondere die für kosmetische Zwecke üblicherweise verwendeten niederen einwertigen Alkohole mit 1 bis 4 Kohlenstoffatomen wie zum Beispiel Ethanol und Isopropanol, oder mehrwertige Alkohole wie Glycerin, Ethylenglykol oder Propylenglykol enthalten sein. Das erfindungsgemäße Mittel liegt vorzugsweise bei einem pH-Wert vor, bei welchem die Fettsäure in Form der freien Säure vorliegt, insbesondere bei einem pH im Bereich von 3 bis 7, besonders bevorzugt von 4 bis 6,5, ganz besonders bevorzugt von 4,5 bis 6.

Es wurde gefunden, dass sich die Wirkungen der drei Bestandteile potenzieren, die Bildung von gesundem Haar besonders gefördert wird, Haarveränderungen und Haarausfall entgegengewirkt wird, die Erholung bei reversiblen Haarausfällen gefördert wird und irreversibler Haarausfall gebremst wird.

### Zusätzlicher Wirkstoff

Eine weitere Wirkungssteigerung kann durch die Verwendung von zusätzlichen Stoffen erzielt werden, welche an den gleichen oder an zusätzlichen, für das Haarwachstum essentiellen Stellen eingreifen, z.B. weiteren durchblutungsfördernden Stoffen oder Stoffen, welche gefäßstabilisierende oder gefäßerhaltende Eigenschaften haben. In einer besonderen Ausführungsform enthält das erfindungsgemäße Haartonikum daher mindestens einen zusätzlichen Wirkstoff, welcher an mindestens einer für das Haarwachstum essentiellen Stelle eingreift, insbesondere einen zusätzlichen durchblutungsfördernden Stoff. Eine durchblutungsfördernde Substanz im Sinne der Erfindung ist ein Stoff, welcher nach Aufbringung auf die Kopfhaut deren Durchblutung steigert und dadurch insbesondere die Nährstoffversorgung des Haarorgans verstärkt.

Die zusätzlichen Wirkstoffe sind vorzugsweise in einer Menge von 0,001 bis 2, besonders bevorzugt von 0,01 bis 1 Gew.% enthalten. Geeignet sind die verschiedenen Tocopherole (alpha-, beta-, gamma- oder delta-Tocopherol), vorzugsweise alpha-Tocopherol sowie deren Ester, insbesondere Vitamin E (Tocopherylacetat), Tocopherylsuccinat, -nicotinat oder -poly(oxyethylen)succinat. Geeignet sind auch Nicotinsäure und deren Derivate, insbesondere Nicotinamid, Methylnicotinat oder Tocopherylnicotinat, sowie Kampher, Gelee Royal und durchblutungsfördernde Pflanzenextrakte wie Brennesselextrakt (Urtica Dioica), Roßkastanienextrakt (Aesculus Hippocastanum), Arnikaextrakt (Arnica Montana), Heublumenextrakt, Fichtennadelextrakt (Pinus) oder Swertia Chirata.

Das erfindungsgemäße Haartonikum kann darüber hinaus die für Haarbehandlungsmittel üblichen Zusatzbestandteile enthalten, z.B. Parfümöle in einer Menge von 0,01 bis 0,5 Gew.%; Trübungsmittel, wie z.B. Ethylenglykoldistearat, in einer Menge von etwa 0,2 bis 5 Gew.%; Tenside insbesondere Emulgatoren; Lösungsvermittler; Konservierungsmittel wie z.B. Parabene in einer Menge von 0,01 bis 1 Gew.%; Puffersubstanzen, wie z.B. Natriumcitrat oder Natriumphosphat, in einer Menge von 0,1 bis 1 Gew.%; Pflegestoffe, wie z.B. haar- oder hautpflegende Pflanzen- und Kräuterextrakte, Protein- und Seidenhydrolysate, Lanolinderivate, in einer Menge von 0,1 bis 5 Gew.%; physiologisch verträgliche Silikonderivate, wie z.B. flüchtige oder nicht-flüchtige Silikonöle oder hochmolekulare Siloxanpolymere in einer Menge von 0,05 bis 20 Gew.%; Lichtschutzmittel, Antioxidantien, Radikalfänger, Antischuppenwirkstoffe, in einer Menge von etwa 0,01 bis 2 Gew.%; Haarglanzgeber, Vitamine, Kämmbarkeitsverbesserer und rückfettende Agenzien.

Das erfindungsgemäße Haartonikum wird angewendet, indem es in einer zur Erzielung des gewünschten Effektes ausreichenden Menge auf die Kopfhaut aufgetragen und leicht einmassiert wird.

Die nachfolgenden Beispiele sollen den Gegenstand der Erfindung näher erläutern.

### Beispiele

### Beispiel 1: Haartonikum

| | |
|---|---|
| Coffein | 1,0 g |
| Laurinsäure | 0,2 g |
| Biotin | 0,01 g |
| Panthenol | 0,6 g |
| Menthol | 0,25 g |
| Parfüm | 0,2 g |
| PEG-40 Hydrogenated Castor Oil | 0,18 g |
| Tocopherylacetat | 0,05 g |
| Wasser | 45,0 g |
| Ethanol | Ad 100 g |

### Beispiel 2: Haartonikum

| | |
|---|---|
| Coffein | 0,5 g |
| Myristinsäure | 0,4 g |
| Biotin | 0,01 g |
| Panthenol | 0,6 g |
| Menthol | 0,25 g |
| Parfüm | 0,2 g |
| PEG-40 Hydrogenated Castor Oil | 0,18 g |
| Tocopherylacetat | 0,05 g |
| Wasser | 45,0 g |
| Ethanol | Ad 100 g |

### Beispiel 2: Haartonikum

| | |
|---|---|
| Coffein | 1,0 g |
| Laurinsäure | 0,1 g |
| Myristinsäure | 0,1 g |
| Biotin | 0,01 g |
| Panthenol | 0,3 g |
| Menthol | 0,1 g |
| Parfüm | 0,2 g |
| PEG-40 Hydrogenated Castor Oil | 0,18 g |
| Tocopherylacetat | 0,05 g |
| Wasser | 45,0 g |
| Ethanol | Ad 100 g |

### Beispiel 4: Wirksamkeitsstudie

Die Wirksamkeit des erfindungsgemäßen Mittels wurde in einer Studie überprüft. Hierzu wurde ein Gruppe von 18 männlichen Probanden (Gruppe A(M)) und eine Gruppe von 19 weiblichen Probanden (Gruppe A(W)) mit je einem wirkstoffhaltigen Produkt (4A(M) bzw. 4A(W)) und eine Gruppe von 17 männlichen Probanden (Gruppe B(M)) und eine Gruppe von 18 weiblichen Probanden (Gruppe B(W)) mit je einem wirkstofffreien Placebo (4B(M) bzw. 4B(W)) behandelt. Sämtliche Probanden wiesen androgene Alopecie II. und III. Grades der Hamilton-Skala auf.

Die Anwendung erfolgte bei allen Probanden über insgesamt 90 Tage, davon die ersten 45 Tage täglich und die folgenden 45 Tage zweimal wöchentlich.

| Wirkstoffhaltiges Produkt 4A (M): | |
|---|---|
| Laurinsäure | 0,2 g |
| Coffein | 1,0 g |
| Biotin | 0,01 g |
| Panthenol | 0,6 g |
| Tocopherylacetat | 0,05 g |
| Pflanzenextrakt⁽¹⁾ | 0,1 g |
| Menthol | 0,25 g |
| PEG-40 Hydrogenated Castor Oil | 0,18 g |
| PEG-35 Castor Oil | 0,46 g |
| Parfüm | 0,5 g |
| Wasser | ad 100 g |
| Ethanol, 95%ig | 55 g |

| | |
|---|---|
| ⁽¹⁾ Extrapon® 5-UK neu (Dragoco): Wasser, Propylenglykol, Ethoxydiglykol, Betula Alba, Equisetum Urtica, Salvia Officinalis, Dioica Arvense, Rosmarinos Officinalis, Achillea Millefollium, Inositol, Calciumpantothenat, Natriumbenzoat, Kaliumsorbat. | |

| Wirkstoffhaltiges Produkt 4A (M): | |
|---|---|
| Laurinsäure | 0,2 g |
| Coffein | 0,9 g |
| Biotin | 0,01 g |
| Panthenol | 0,6 g |
| Tocopherylacetat | 0,05 g |
| Multivitamin-Kräuterkomplex Soluvit® Richter | 0,1 g |
| Menthol | 0,25 g |
| PEG-40 Hydrogenated Castor Oil | 0,18 g |
| PEG-35 Castor Oil | 0,46 g |
| Parfüm | 0,2 g |
| Wasser | ad 100 g |
| Ethanol, 95%ig | 55 g |

Das Vergleichsprodukt 4B (M) hatte folgende Zusammensetzung:

| | |
|---|---|
| Parfüm | 0,5 g |
| Ethanol, 95%ig | 55 g |
| Wasser | Ad 100 g |

Das Vergleichsprodukt 4B (M) hatte folgende Zusammensetzung:

| | |
|---|---|
| Parfüm | 0,2 g |
| Ethanol, 95%ig | 55 g |
| Wasser | Ad 100 g |

Untersuchungen des Haarausfalls wurden vor Behandlungsbeginn (t0), nach 45 Tagen (t45) und nach 90 Tagen (t90) durchgeführt. Die Untersuchungen erfolgten nach drei Methoden: einer objektiven Beurteilung durch den die Tests durchführenden und auswertenden Wissenschaftler, einer subjektiven Beurteilung durch den jeweiligen Probanden selber und einer quantitativen Standard-Messung (Pull-Test).

Die subjektiven und die objektiven Beurteilungen erfolgten nach einer Skala von 0 bis 6:
0: kein Haarausfall
2: leichter Haarausfall
4: mittlerer/moderater Haarausfall
6: starker Haarausfall

Die quantitative Standard-Messung erfolgte nach dem sogenannten Pull-Test, beschrieben in Cosmesi Dermatologica, N. 65, Aprile-Giugno 1998, Seite 139. Hierbei wird eine Haarsträhne von ca. 60 Haaren in Höhe der Scheitelregion 2-3 cm über dem äußeren Gehörgang zwischen Daumen und Zeigefinger genommen und dabei eine konstante Traktion bis zum distalen Teil des Haares ausgeübt. Dann wird die Anzahl der ausgerissenen Haare gezählt. Durch den Pull-Test wird der Anteil der nur noch lose in der Kopfhaut verankerten Haare ermittelt. Durch mikroskopische Untersuchung der Haarwurzel kann der Haarzustand (telogen oder anagen) bestimmt werden. Der Test ist indikativ für *Telogen effluvium*, *Anagen effluvium*, Bruch des Haarwurzelschaftes, androgenetischen Haarausfall, örtlich lokalisierten und diffusen Haarausfall.

Die Ergebnisse sind in der folgenden Tabelle zusammengefasst:

| | | A(W) | A(M) | B(W) | B(M) |
|---|---|---|---|---|---|
| Haarausfall (objektiv) | t0 | 3,4 | 3,6 | 3,2 | 3,6 |
| | t45 | 3,0 | 3,3 | 3,2 | 3,6 |
| | t90 | 2,4 | 2,7 | 3,1 | 3,4 |
| | Δ⁽¹⁾ | 73,7% | 72,2% | 11,1% | 17,6% |
| Haarausfall (subjektiv) | t0 | 3,4 | 3,8 | 3,1 | 3,4 |
| | t45 | 2,8 | 3,1 | 3,1 | 3,3 |
| | t90 | 2,3 | 2,6 | 2,9 | 3,1 |
| | Δ⁽¹⁾ | 78,9% | 77,8% | 16,7% | 23,5% |
| Pull-Test | t0 | 10,9 | 11,7 | 10,0 | 11,5 |
| | t45 | 9,7 | 10,1 | 9,9 | 11,3 |
| | t90 | 6,8 | 7,2 | 9,4 | 10,2 |
| | Δ⁽²⁾ | 37,6% | 38,5% | 6,0% | 11,3% |

| | | | | | |
|---|---|---|---|---|---|
| Δ⁽¹⁾: Anzahl der Probanden in %, bei denen sich der Haarausfall verringert hat | | | | | |
| Δ⁽²⁾: Verringerung der Anzahl nur noch lose in der Kopfhaut verankerter Haare in % | | | | | |

Alle Ergebnisse waren nach statistischer Auswertung signifikant. Die Ergebnisse zeigen eine deutliche Verringerung des Haarausfalls sowie eine deutliche Stärkung der Verankerung der Haare in der Kopfhaut.

## Patentansprüche

1. Kosmetisches Mittel mit einem Gehalt an
(A) mindestens einer Fettsäure oder deren Salz, ausgewählt aus Laurinsäure, Myristinsäure und Isomyristinsäure und
(B) Biotin und
(C) Coffein.

2. Mittel nach Anspruch 1, **dadurch gekennzeichnet, dass** die Fettsäure (A) Laurinsäure oder Myristinsäure oder ein Gemisch dieser beiden Säuren ist.

3. Mittel nach einem der vorhergehenden Ansprüche,
**dadurch gekennzeichnet, dass** es mindestens eine zusätzliche durchblutungsfördernde Substanz enthält.

4. Mittel nach Anspruch 3, **dadurch** gekennnzeichnet, dass die durchblutungsfördernde Substanz ausgewählt ist aus Tocopherolen und deren Estern, Nicotinsäure und deren Estern, Nicotinamid, Kampher, Gelee Royal und durchblutungsfördernden Pflanzenextrakten.

5. Mittel nach einem der vorhergehenden Ansprüche,
**dadurch gekennzeichnet, dass** es in Form eines Haartonikums vorliegt. Kosmetisches Mittel gemäß Anspruch 1 zur Verwendung zur Vorbeugung oder Behandlung von Haarausfall.

6. Kosmetisches Mittel gemäß Anspruch 1 zur Verwendung zur Vorbeugung oder Behandlung von Haarausfall.

## Claims

1. Cosmetic agent with a content of
(A) at least one fatty acid or salt thereof, chosen from lauric acid, myristic acid and isomyristic acid and
(B) biotin and
(C) caffeine.

2. Agent according to Claim 1, **characterized in that** the fatty acid (A) is lauric acid or myristic acid or a mixture of these two acids.

3. Agent according to one of the preceding claims, **characterized in that** it comprises at least one additional circulation-promoting substance.

4. Agent according to Claim 3, **characterized in that** the circulation-promoting substance is chosen from tocopherols and esters thereof, nicotinic acid and esters thereof, nicotinamide, camphor, royal jelly and circulation-promoting plant extracts.

5. Agent according to one of the preceding claims, **characterized in that** it is in the form of a hair tonic.

6. Cosmetic agent according to Claim 1 for use for preventing or treating hair loss.

## Revendications

1. Composition cosmétique ayant une teneur en
(A) au moins un acide gras ou un sel de celui-ci, choisi parmi l'acide laurique, l'acide myristique et l'acide isomyristique et
(B) biotine et
(C) caféine.

2. Composition selon la revendication 1, **caractérisée en ce que** l'acide gras (A) est l'acide laurique ou l'acide myristique ou un mélange de ces deux acides.

3. Composition selon l'une quelconque des revendications précédentes, **caractérisée en ce qu'**elle contient au moins une substance supplémentaire favorisant l'irrigation sanguine.

4. Composition selon la revendication 3, **caractérisée en ce que** la substance favorisant l'irrigation sanguine est choisie parmi les tocophérols et leurs esters, l'acide nicotinique et ses esters, le nicotinamide, le camphre, la gelée royale et des extraits de plantes favorisant l'irrigation sanguine.

5. Composition selon l'une quelconque des revendications précédentes, qui se trouve sous la forme d'une lotion capillaire tonifiante.

6. Composition cosmétique selon la revendication 1, pour utilisation dans la prévention ou le traitement de la chute des cheveux.
